(19) Europäisches Patentamt
European Patent Office
Office européen des brevets

(11) Veröffentlichungsnummer: **0 571 883 A1**

(12) **EUROPÄISCHE PATENTANMELDUNG**

(21) Anmeldenummer: **93108107.9**

(51) Int. Cl.⁵: **C08G 65/40**, C07C 49/83

(22) Anmeldetag: **18.05.93**

(30) Priorität: **26.05.92 DE 4217347**

(43) Veröffentlichungstag der Anmeldung:
**01.12.93 Patentblatt 93/48**

(84) Benannte Vertragsstaaten:
**BE CH DE FR GB IT LI NL**

(71) Anmelder: **HOECHST AKTIENGESELLSCHAFT**

**D-65926 Frankfurt(DE)**

(72) Erfinder: **Wilharm, Peter, Dr.**
**Hans-Fischer-Strasse 4**
**W-8906 Gersthofen(DE)**
Erfinder: **Weller, Thomas, Dr.**
**Am Jungstück 42**
**W-6500 Mainz(DE)**
Erfinder: **Meier, Michael, Dr.**
**Franz-Rücker-Allee**
**W-6000 Frankfurt/Main(DE)**

# (54) Polyarylenether mit Xanthoneinheiten, Verfahren zu ihrer Herstellung und ihre Verwendung.

(57) Polyarylenether, die Xanthoneinheiten enthalten und eine sehr hohe Temperaturbeständigkeit aufweisen, können aus preisgünstigen, gut zugänglichen Monomeren durch Polykondensation in basischem Medium unter im wesentlichen wasserfreien Bedingungen in Anwesenheit oder Abwesenheit eines aromatischen Lösemittels bei Temperaturen von 150 bis 400 °C hergestellt werden. Hierbei kann man sowohl von Xanthon-Monomeren als auch von hydroxyhalogen-substituierten Benzophenon-Verbindungen ausgehen.

EP 0 571 883 A1

Die Erfindung betrifft Polyarylenether mit Xanthoneinheiten, ein Verfahren zu ihrer Herstellung aus preisgünstigen Ausgangsmaterialien und ihre Verwendung zur Herstellung von Formkörpern.

Polyarylenether sind seit langem bekannt und zeichnen sich durch wertvolle Gebrauchseigenschaften, wie hohe Temperaturbeständigkeit und gute elektrische und mechanische Eigenschaften aus. Insbesondere die teilkristallinen Polyarylenetherketone zeigen zusätzlich gute Beständigkeit gegenüber Lösemittel und Chemikalienangriff.

Zur Herstellung von Polyarylenetherketonen sind verschiedentlich sogenannte AB-Monomere, d.h. mit sich selbst kondensierbare Monomere, verwendet worden. Solche Monomere besitzen den Vorteil, daß die Erzielung hoher Molekulargewichte nicht von der Einhaltung einer genauen Stöchiometrie abhängt, wie bei der Reaktion von sogenannten AA- mit BB-Monomeren, z. B. bei der Reaktion von Bisphenolen mit aktivierten Bishalogen-Aromaten.

Zur Herstellung von Polyarylenetherketonen mit hohem Molekulargewicht wird im allgemeinen vorteilhaft das nucleophile Verfahren herangezogen, weil damit struktureinheitlichere Polymere als mit dem elektrophilen Verfahren erzielbar sind.

Es ist bereits bekannt, daß sich Hydroxyhalogen-Aromaten (AB-Monomere), die mindestens zweikernig sind und deren Chlor- oder Fluor-Abgangsgruppen durch ortho- oder para-ständige elektronenabziehende Gruppen aktiviert sind, unter Bedingungen der nucleophilen Polykondensation zu Polyarylenetherketonen umsetzen lassen. Beispielsweise wird durch eine Cu-katalysierte nucleophile Polykondensation von 4-Chlor-4'-hydroxy-benzophenon das entsprechende Polyarylenetherketon erhalten (EP-B 0 182 648). Diese Route ist attraktiv, weil die verwendeten Chlor-Monomere preisgünstig und einfach herstellbar sind. Nachteilig ist jedoch, daß die dort verwendeten Chlor-Monomere im Vergleich zu den entsprechenden Fluor-Monomeren reaktionsträge sind und im allgemeinen die Anwendung von Cu-Verbindungen als Katalysatoren benötigen. Diese Cu-Katalysatoren sind aus den entstehenden Polyarylenetherketonen nur schwierig, wenn überhaupt, vollständig zu entfernen und verschlechtern die Schmelzestabilität beträchtlich.
Weiterhin müssen bei der Aufarbeitung entstehende Cu-haltige Abwässer aus Umweltschutzgründen aufwendig gereinigt werden.

Weiterhin sind heterocyclische AB-Monomere, die sich unter den Bedingungen der nucleophile Polykondensation umsetzen lassen, bekannt (DE-C 20 38 240). Hier wird die Polykondensation von 2-Hydroxy-6-chlor-dibenzooxathiin beschrieben. Diese Polymere sind zwar sehr temperaturbeständig und weisen hohe Glasübergangstemperaturen auf, zeigen aber eine hohe Wasseraufnahme und geringe Lösemittelresistenz.

Bekannt ist auch, daß Polyarylenetherketone mit Xanthon-Einheiten aus entsprechenden polymeren Chlor-hydroxy-benzophenonen herstellbar sind (DE-C 18 06 419). Nachteilig ist, daß die als Ausgangsmaterialien verwendeten Polyketone nur aufwendig herstellbar sind und daß die erhaltenen Polymere, wie eigene Versuche zeigen, nicht schmelzeverarbeitbar sind.

Weiterhin wird ein Verfahren zur Herstellung von linearen, hochmolekularen, thermoplastischen Polyetherketonen beschrieben, bei dem u. a. auch Xanthon-Monomere vom AA-Typ mit Bisphenolen vom BB-Typ umgesetzt werden (GB-B 1 238 124). Jedoch können im allgemeinen Polymere, wie sie in der vorliegenden Erfindung beschrieben werden, nicht hergestellt werden. Weiterhin nachteilig ist, daß die Stöchiometrie sehr genau eingehalten werden muß, um Produkte mit hohem Molekulargewicht zu erhalten.

Bekannt sind auch Polymere mit wiederkehrenden Einheiten der Formel

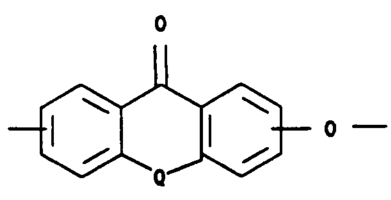

worin
Q -$C(R^1)_2$-, -CO-, -O-, -S-, -$NR_2$-, -$Si(R^3)_2$-, -$P(O)R^4$- mit $R^1$ gleich Wasserstoff, Alkylgruppen mit einer Kohlenstoffzahl von 1 bis 5 oder Phenylgruppen, $R^2$, $R^3$, $R^4$ Alkylgruppen mit einer Kohlenstoffzahl von 1 bis 5 oder Phenylgruppen bedeuten (JP-A 60-71635). Diese Polymere werden durch Selbstkondensation der entsprechenden Hydroxy-Halogen-Monomere erhalten. In der Aufzählung der geeigneten Phenole wird auch ein Xanthon-Monomer, 3-Fluor-7-hydrory-xanthen-5-on, angeführt, jedoch läßt sich dieses, wie eigene Versuche gezeigt haben, unter Anwendung der in den Beispielen genannten Versuchsbedingungen nicht zum entsprechenden aromatischen Polyether umsetzen.

Es bestand daher die Aufgabe, lineare Polyarylenether mit sehr hoher Temperaturbeständigkeit aus preisgünstigen, gut zugänglichen Monomeren bereitzustellen.

Die Erfindung betrifft somit Polyarylenether mit den wiederkehrenden Einheiten der Formeln

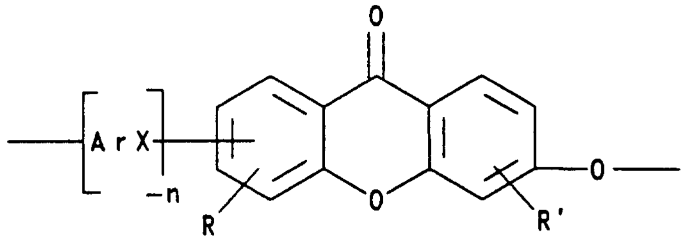

(Ia)

und/oder

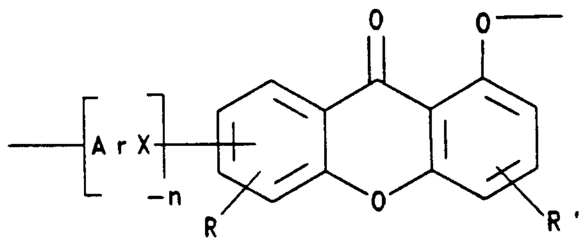

(Ib)

worin

R und R' gleich oder verschieden sind und Wasserstoff, Alkyl-, Alkoxy-, Aryl-, Aryloxy-Gruppen, benzanellierte Reste, vorzugsweise Wasserstoff, Methyl-, Phenyl-, Methoxy-, Phenoxy-Gruppen, insbesondere aber Wasserstoff bedeuten,

Ar ein divalenter aromatischer Rest, vorzugsweise eine Phenylen-Gruppe,

X eine direkte Bindung, -O- oder -S- und

n Null oder eins ist.

Die Herstellung von Polymeren und Copolymeren mit wiederkehrenden Einheiten der Formel (Ia) oder (Ib) umfaßt (a) die nucleophile Polykondensation unter im wesentlichen wasserfreien Bedingungen in Anwesenheit oder Abwesenheit eines polaren, apotischen aromatischen Lösemittels (i) wenigstens eines Hydroxyhalogen-substituierten Xanthons der Formel (IIa) oder (IIb)

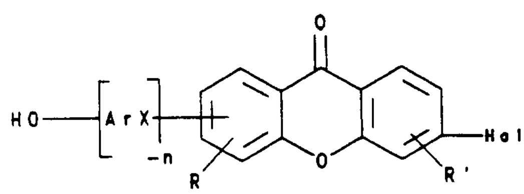

(IIa)

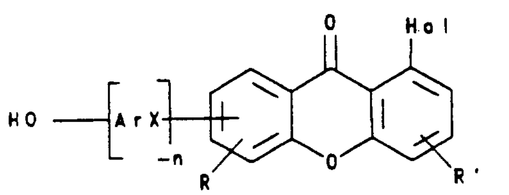

(IIb)

in welcher

R, R', Ar, X und n die obengenannte Bedeutung haben und

Hal für Chlor- oder Fluor-Gruppen, insbesondere für Chlor-Gruppen steht, oder (ii) wenigstens einer

Hydroxyhalogen-substituierten Benzophenon-Verbindung der Formel (IIIa) oder (IIIb)

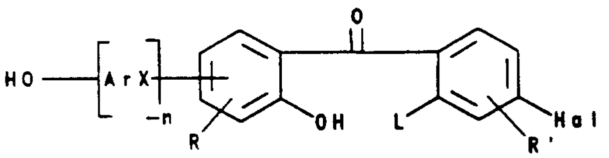

(IIIa)

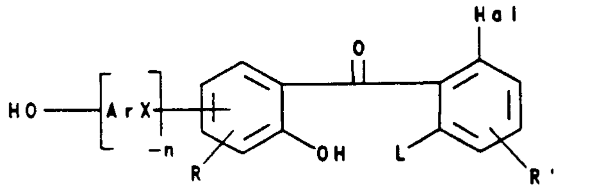

(IIIb)

worin
R, R', Ar, X, Hal und n die obengenannte Bedeutung haben und
L eine durch intramolekulare nucleophile Substitution verdrängbare Fluchtgruppe, vorzugsweise eine Chlor- oder Fluor-Gruppe, insbesondere eine Chlor-Gruppe bedeutet,
oder (b) die nucleophile Polykondensation unter basischen Bedingungen wenigstens einer Hydroxy-halogen-Verbindung gemäß (a) zusammen mit wenigstens einer Hydroxy-halogen-Verbindung der Formel (IV)

HO - Ar' - Hal (IV)

worin Ar' ein divalenter aromatischer Rest mit 12 bis 26, vorzugsweise 12 bis 20 C-Atomen ist, der mindestens eine Keto- oder Sulfonyl-Gruppe enthält, die ortho- oder para-ständig zur Hal-Gruppe steht und Hal für eine Chlor- oder Fluor-Gruppe steht, die sich am äußersten aromatischen Kern befindet.

Xanthon-Verbindungen gemäß Formel (Ia) oder (Ib) sind teilweise bekannt oder können nach bekannten Verfahren hergestellt werden. Zu ihrer Herstellung z. B. durch intramolekulare Substitution können beispielsweise Benzophenon-Verbindungen gemäß Formel (IIIa) oder (IIIb) als Ausgangsverbindungen herangezogen werden (Chem. Pharm. Bull. 38, 1266 (1990)).

Weiterhin können Xanthon-Verbindungen gemäß Formel (IIa) oder (IIb) durch elektrophile Cyclisierung hergestellt werden (Comprehensive Heterocyclic Chemistry, Vol. 3, S, 647 ff. und S. 737 ff., Pergamon Press (1984), ferner R.C. Elderfield, Ed., Heterocyclic Chemistry, Vol. II, S. 419 ff, Wiley (1951)).

Geeignete Xanthon-Verbindungen gemäß Formel (IIa) oder (IIb) sind beispielsweise

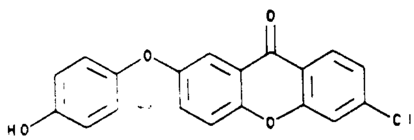
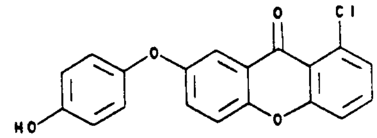

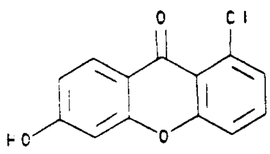
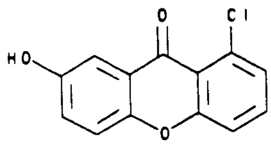
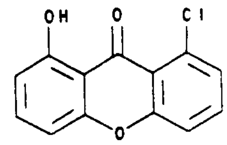

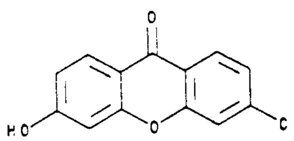
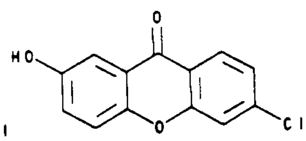
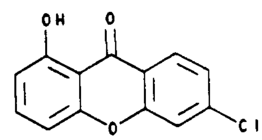

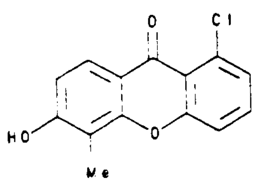
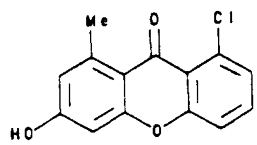
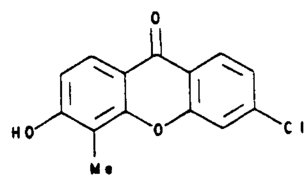

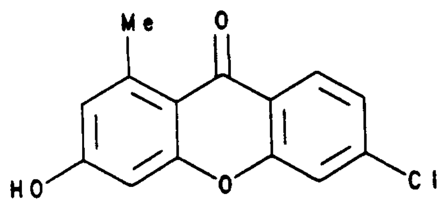

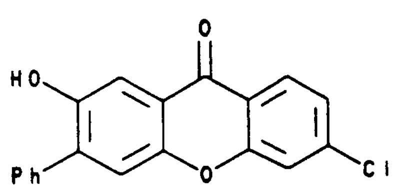
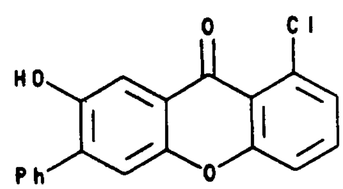

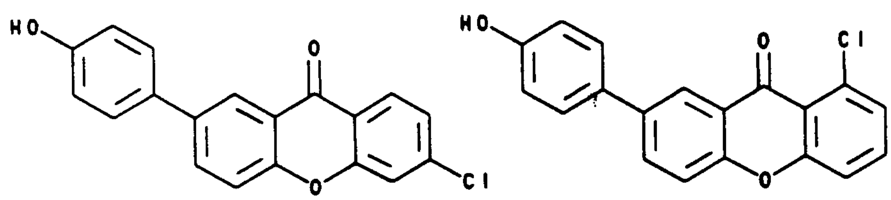

und die entsprechenden kernfluorierten Verbindungen. In den Formeln steht Me für eine Methyl- und Ph für eine Phenyl-Gruppe. Die Hydroxyhalogen-substituierten Benzophenon-Verbindungen der Formel (IIIa) oder (IIIb) können auf bekannte Weise, beispielsweise durch Fries'sche-Umlagerung der entsprechend substituierten aromatischen Ester oder durch Acylierung der entsprechenden Phenole oder Phenol-methylether gewonnen werden.

Geeignete Benzophenon-Verbindungen gemäß Formel (IIIa) oder (IIIb) sind beispielsweise

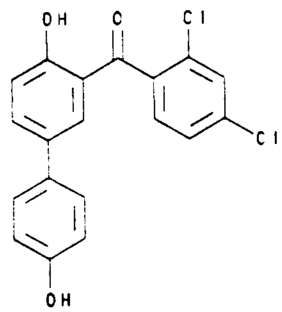

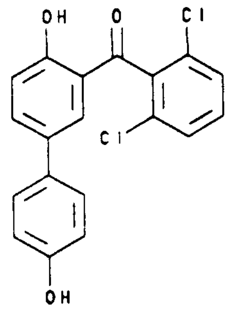

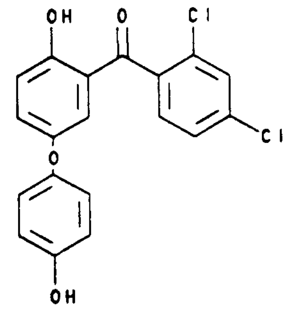

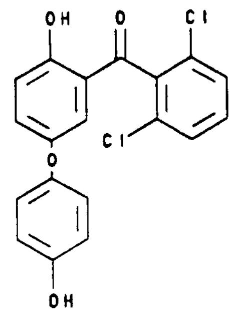

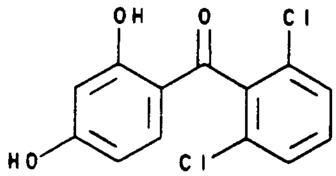 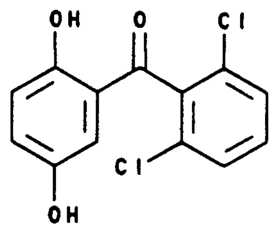 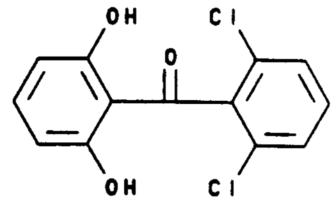

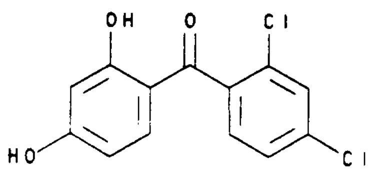 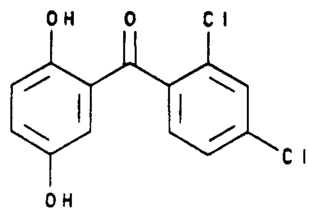 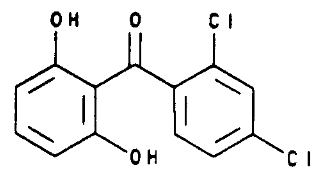

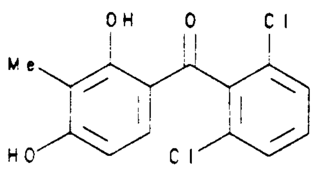 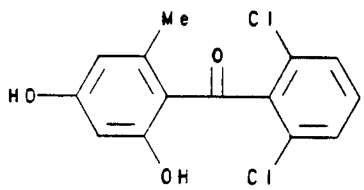 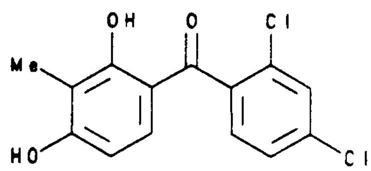

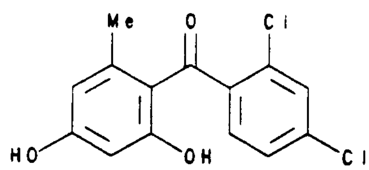

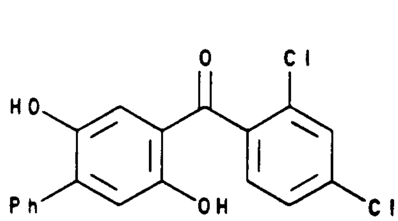 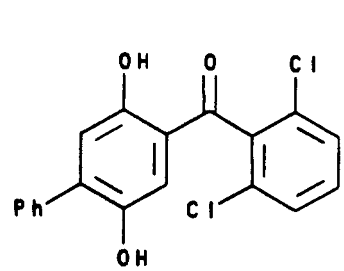

und die entsprechenden kernfluorierten Verbindungen, wobei Me und Ph wie vorstehend definiert sind. Zur Herstellung von Polyarylenethern, die wiederkehrende Einheiten der Formel (Ia) oder (Ib) enthalten, können Verbindungen gemäß (IIa), (IIb), (IIIa) oder (IIIb) oder Mischungen daraus verwendet werden.

Weiterhin können zur Herstellung von Copolymeren Monohydroxy-monohalogen-Verbindungen gemäß Formel (IV) eingesetzt werden. Beispiele für solche Comonomere sind Verbindungen gemäß Formeln (V) oder (VI)

HO - Ph - T - Ph' - Hal (V),

HO - Ph - Y - Ar'- Z - Ph' - Hal (VI),

worin Ph und Ph' gleich oder verschieden sind und für eine unsubstituierte oder eine mit Methyl- oder Phenyl-Gruppen substituierte Phenylen-Gruppe stehen, Ar' als divalenter Rest eine Phenylen- oder Biphenyl-Gruppe bedeutet, T ist eine Keto- oder Sulfon-Gruppe, Y ist eine direkte Bindung oder eine Ether-, Keto- oder Sulfon-Gruppe, Z ist eine Keto- oder Sulfon-Gruppe, Hal ist Halogen, vorzugsweise Chlor oder Fluor, das jeweils ortho- oder para-ständig zu Z steht.

Einsetzbare Monohydroxymonohalogen-Verbindungen sind beispielsweise 4-Chlor-4'-hydroxybenzophenon, 4-(4'-Chlorbenzoyl)-4'-hydrorybiphenyl, 4-(4'-Chlorbenzoyl)-4'-hydroxydiphenylether, 4-Chlor-4'-hydroxy-terephthalophenon, 4-Chlor-4'-hydroxy-isophthalophenon, 4-(4'-Hydroxybenzoyl)-4'-(4-chlorbenzoyl)biphenyl, 4-Chlor-4'-hydroxy-diphenylsulfon, 4-Hydroxy-4'-(4'-chlorophenylsulfonyl)biphenyl, 4-(4-Hydroxyphenylsulfonyl)-4'-(4-chlorophenylsulfonyl)biphenyl, 4-Hydroxy-4'-(4'-chlorophenylsulfonyl)-diphenylether, 4-Hydroxy-3,5-dimethyl-4'-chloro-benzophenon, 4-Hydroxy-3,5-diphenyl-4'-chlorobenzophenon, die entsprechenden Fluor-substituierten Verbindungen oder Mischungen aus den genannten Verbindungen.

Bevorzugt sind 4-Chlor-4'-hydroxybenzophenon, 4-Chlor-4'-hydroxy-terephthalophenon, 4-Chlor-4'-hydroxy-isophthalophenon, 4-Chlor-4'-hydroxy-diphenylsulfon, 4-Hydroxy-3,5-dimethyl-4'-chloro-benzophenon, deren Fluoranaloge und Mischungen daraus.

Die Herstellung der Polymere gemäß der Erfindung durch nucleophile Polykondensation erfolgt auf bekannte Weise unter Bedingungen - Verfahrensbedingungen, Lösemittel und Zusätze - wie sie zur Herstellung anderer Polyarylenether bekanntgeworden sind (EP 0 001 879, EP 0 193 187, US 4 108 837, US 4 175 175 und in "Comprehensive Polymer Science", Band 5, Seite 483 ff. und Seite 561 ff., Hrsg. G. Allen, Pergamon Press 1989 und in Polymer 22, 1096 (1981)).

Ein besonderer Vorteil des Verfahrens gemäß der Erfindung liegt darin, daß im allgemeinen Chlor- statt der teureren Fluor-Monomere verwendet werden können. Die Reaktivität bei der nucleophilen Substitution von Chlor-Gruppen in ortho- oder para-Stellung bezüglich der Keto-Gruppe in den Xanthon-Monomeren ist im Vergleich zu den entsprechenden Chlor-benzophenon-Monomeren deutlich erhöht.

Bevorzugt ist eine Ausführungsform des Verfahrens, bei der Xanthon-Verbindungen (IIa) oder (IIb) in einem der Polykondensation vorgelagerten Schritt "in situ" aus Benzophenon-Verbindungen gemäß Formel (IIIa) oder (IIIb) erzeugt werden. Vorteilhaft ist dabei, daß die vorherige Isolierung der Xanthone entfällt.

Zur "in situ"-Herstellung der Xanthon-Verbindungen eignet sich das sogenannte "Azeotrop"-verfahren. Dabei wird das bei der Phenolat-Synthese entstehende Wasser durch einen Azeotropbildner kontinuierlich aus dem Reaktionsgemisch entfernt.

Geeignete Wasser-Azeotropbildner sind alle Substanzen, die im Bereich der Reaktionstemperatur bei Normaldruck sieden und sich mit dem Reaktionsgemisch mischen lassen, ohne chemische Reaktionen einzugehen. Derartige Azeotropbildner sind beispielsweise: Chlorbenzol, Toluol, Xylol, langkettige aliphatische Kolenwasserstoffe, Cyclohexan.

Wenn als Polykondensations-Lösemittel Diphenylsulfon, Xanthon, Benzophenon oder Mischungen daraus verwendet werden, wird eine Ausführungsform des Verfahrens bevorzugt, bei der die "in situ"-Herstellung der Xanthon-Verbindungen und die Polykondensation durch stufenweise Erhöhung der Reaktionstemperatur nacheinander ohne Zugabe eines Azeotropbildners erfolgt.

Die Polykondensation wird in Gegenwart einer anorganischen Base durchgeführt. Erfindungsgemäß geeignete Verbindungen sind Alkali-Hydroxide, -Carbonate, - Hydrogencarbonate, -Fluoride.
Bevorzugt sind die Carbonate und Hydrogencarbonate des Natriums und Kaliums wie Natriumcarbonat, Natriumhydrogencarbonat, Kaliumcarbonat, Kaliumhydrogencarbonat, und Mischungen daraus.

Die Gesamtmenge an Base ist im allgemeinen so bemessen, daß pro Mol Hydroxylgruppen wenigstens ein Mol Metallatome, vorzugsweise 1,0 bis 1,2 Mol Metallatome vorhanden sind.

Wenn als Base Alkalimetall-Hydroxid angewendet wird, so wird diese vorzugsweise in einem der Polykondensation vorgelagerten Schritt mit den phenolischen OH-Gruppen der Monomere gemäß Formel (IIa), (IIb), (IIIa) oder (IIIb) zu den betreffenden Alkalimetall-Phenolaten umgesetzt. Die isolierten Phenolate sollen eine Korngröße von kleiner als 0,5 mm, vorzugsweise 1 bis 350 $\mu$m aufweisen.

Die Phenolat-Bildung bei Anwendung von Alkalimetall-Hydroxiden soll bei niedrigen Temperaturen, vorzugsweise bei Temperatur von kleiner als 100 °C, erfolgen, um eine Hydrolyse der Halogengruppen zu vermeiden. Beispielsweise werden die Alkalimetall-Phenolate zweckmäßigerweise aus in Wasser/Alkohol-Gemischen unter Zugabe von stöchiometrischer Mengen Alkalimetall-Hydroxid und anschließendem Entfernen des Lösemittels bei Temperaturen von kleiner als 100 °C gewonnen.

Die Herstellung der Polymere gemäß der Erfindung erfolgt entweder in einem polaren, aprotischen Lösemittel oder in Abwesenheit eines Lösemittels in der Schmelze. Für die Durchführung der Polykondensation geeignete Lösemittel sind beispielsweise aromatische Sulfoxide und Sulfone, Diphenylsulfon, Ditolylsulfon, Dibenzothiophen-S,S-dioxid, 4-Phenylsulphonyl-biphenyl und cyclische Harnstoffe, wie 1,3-Dimethyl-3,4,5,6-tetrahydro-2-pyrimidon, aromatische Ketone, wie Xanthon, Thioxanthon, Benzophenon, Fluorenon, 4-Phenoxy-benzophenon, Isophthalophenon.

Besonders bevorzugt sind Diphenylsulfon, Xanthon, Benzophenon und Mischungen daraus.

Bei der Verwendung der aromatischen Sulfone als Lösungsmittel werden die Basen in gemahlener und getrockneter Form eingesetzt, wobei die Korngröße kleiner als 0,4 mm, vorzugsweise von 1 bis 350 µm betragen soll.

Die polaren, aprotischen Lösemittel werden in solchen Mengen angewendet, daß die Reaktionslösungen einen Feststoffgehalt von 5 bis 50 Gew.-% vorzugsweise von 20 bis 45 Gew.-% aufweisen.

Um hohe Molekulargewichte zu erzielen, ist es wichtig, daß wahrend der Polykondensationsreaktion im wesentlichen wasserfreie Bedingungen in der Reaktionsmischung sichergestellt werden. Bei der Polykondensationsreaktion entstehendes Reaktionswasser ist daher sofort aus dem System zu entfernen, um eine Hydrolyse der vorzugsweise Chlor-haltigen Monomere zu vermeiden. Dies kann mit Hilfe eines Azeotropbildners, Anlegen von vermindertem Druck oder vorzugsweise durch Einleiten oder Überleiten eines Stickstoffstroms und Abdestillieren erfolgen.

Die untere Grenze der Reaktionstemperatur ist durch die Schmelztemperatur mindestens einer der Komponenten oder des Lösemittels und die obere durch die Zersetzungstemperatur der Kondensationspartner, des Lösemittels oder der entstehenden Polymeren gegeben.

Die angewendete Reaktionstemperatur hängt u.a. von der Reaktivität der Monomere und vom Lösemittel ab und liegt im allgemeinen im Bereich von 150 bis 400 °C, vorzugsweise 200 bis 350 °C.

Die Polykondensation wird vorzugsweise in einer Inertgasatmosphäre z.B. unter einer Stickstoff- oder Argon-Atmosphäre durchgeführt.

Eine Möglichkeit zur Einstellung des gewünschten Molekulargewichts besteht in der Zugabe einer kettenabbrechenden Verbindung (Regler) bei der Polykondensation. Als solche kommen beispielsweise Methylchlorid, 4-Chlordiphenylsulfon, 4-Fluorbenzophenon, 4,4'-Difluor-benzophenon, 1,4-Bis(4-fluorbenzoyl)benzol in Frage.

Die genannten Verbindungen dienen gleichzeitig zur Stabilisierung von eventuell vorhandenen freien Phenolat-Endgruppen.

Die Aufarbeitung der Reaktionslösung kann nach den üblichen, an sich bekannten Verfahren erfolgen. Vorteilhaft wird aus der Schmelze ein feinteiliges Gut erzeugt, welches durch Extraktion mit einem geeigneten Lösemittel (z.B. Aceton, Methanol oder Toluol) von Polykondensationslösemittel (z.B. Xanthon) befreit wird. Ebenso kann zur Extraktion eine abgeschreckte Reaktionslösung, die beispielsweise durch Aufgießen auf eine kalte Metallplatte in einer dünnen Schicht als Festkörper erhalten worden ist, nach feiner Mahlung zur Extraktion benutzt werden. Anschließend können Reste anorganischer Salze durch Extraktion mit Wasser und gegebenenfalls verdünnter Säure entfernt werden.

Die nach dem vorliegenden Verfahren hergestellten Polymere zeigen eine inhärente Viskosität von wenigstens 0,2 dl/g, bevorzugt von 0,2 bis 2,5 dl/g, insbesondere von 0,4 bis 1,8 dl/g.

Die durch das Verfahren hergestellten Polyarylenether zeichnen sich durch eine hohe Temperaturbeständigkeit aus und können vorteilhaft zur Herstellung von Formkörpern wie Fasern, Folien, Spritzgußteilen, Kleb- und Beschichtungsstoffe, sowie als Matrixmaterial für Verbundwerkstoffe (Composites) und Kabelummantelungen verwendet werden. Besonders geeignet sind die Polyarylenether für Anwendungen, bei denen mit hohen Temperaturbelastungen und/oder einer oxidierenden Atmosphäre gerechnet werden muß.

Weiterhin können sie mit anderen Polymeren gemischt und verarbeitet werden und außerdem auch mit Füllstoffen, wie Glasfasern, Kohlefasern, Aramid-Fasern, mineralischen Füll- und Verstärkungsstoffen, wie Calciumcarbonat, Talkum, Magnesiumcarbonat, Glimmer und üblichen Zusatzstoffen, wie Stabilisatoren, Pigmenten und Entformungshilfsmitteln gemischt werden.

Die in den folgenden Beispielen angeführten inhärenten Viskositäten I.V. sind Mittelwerte und wurden nach der Methode von Sorenson et al., die in "Preparative Methods of Polymer Chemistry", Interscience (1968), Seite 49 beschrieben wird, ermittelt: 0,125 g Polymerisat in 25 ml Schwefelsäure (d = 1,84 g/$cm^3$), 25 °C.

Beispiele

Herstellung der Monomere:

1) 2',4'-Dichlor-2,5-dihydroxy-benzophenon: In einem 4-l-Kolben, ausgestattet mit mechanischem Rührer, Rückflußkühler mit aufgesetztem Blasenzähler, thermostatisiertem Heizbad und Innenthermometer wurden 190 g 1,4-Dimethoxybenzol, 138 g 2,4-Dichlorbenzoylchlorid und 2000 ml 1,2-Dichlorbenzol unter Rühren auf 15 °C abgekühlt. Anschließend wurden portionsweise 485 g wasserfreies Aluminiumtrichlorid so zugegeben, daß die Innentemperatur unter 40 °C blieb. Nachdem die Gesamtmenge an Aluminiumtrichlorid zudosiert worden ist, wurde langsam auf 80 °C Innentemperatur aufgeheizt, wobei die Farbe der Reaktionsmischung nach hellgelb umschlug und die Viskosität stark zunahm. Anschließend wurde solange nachgerührt, bis die Gasentwicklung merklich nachließ.

Die Reaktionsmischung wurde auf eine Mischung aus 1 kg Eis, 2 L Wasser und 500 ml 30 %ige Salzsäure gegeben und für 2 Stunden kräftig gerührt. Das ausgefallene Produkt wird abgesaugt, mit Wasser nachgewaschen und bei 60 °C getrocknet.
Ausbeute 60 % (gelbe Kristalle), Schmp. 156 °C.

2) 2-Hydroxy-6-chlor-xanthon: 27 g 2',4'-Dichlor-2,5-dihydroxy-benzophenon, 10,5 g Natriumcarbonat und 100 ml N-Methylpyrrolidon wurden 2 Stunden unter Rühren auf 160 °C erhitzt, wobei unter starker Kohlendioxid-Entwicklung eine intensiv gelbgefärbte Lösung entstand.
Zur Aufarbeitung ließ man auf 80 °C Innentemperatur abkühlen und versetzte unter Rühren tropfenweise mit 100 ml Wasser. Die ausgefallene Kristallmasse wurde abgesaugt und bei 50 °C unter vermindertem Druck getrocknet. Ausbeute 80 %, (farblose Kristalle), Schmp. 268 °C.

Durch analoge Umsetzungen wurden erhalten: 2',4'-Dichlor-2,4-dihydroxy-benzophenon, Schmp. 159 °C; 3-Hydroxy-6-chlor-xanthon, Schmp. 331 °C; 2',4'-Dichlor-2-hydroxy-5-(4-hydroxyphenyl)-benzophenon und 3-Chlor-7-(4-hydroxyphenyl)xanthon.

3) Polykondensation:
Diese wurde in folgender Apparatur durchgeführt:
2-Liter-Doppelmantel-Rührkessel aus V4A-Stahl (Beheizung mit Wärmeträgeröl), ausgestattet mit Bodenablaßventil, Einlaß für Stickstoff-Schutzgas, Rührer aus V4A-Stahl, Thermofühler für Innentemperaturmessung, Abgasrohr mit Tauchung in Wasser (Blasenzähler).
Die Messung der Viskosität der Reaktionslösung erfolgte durch Messung des Drehmoments an der Rührwelle.
Während des gesamten Reaktionsverlaufs wurde ein stetiger Stickstoff-Gasstrom über die Reaktionsmischung geleitet, um das bei der Reaktion entstehende Wasser zu entfernen.

In den Rührkessel wurden eingewogen:
265 g 2-Hydroxy-6-chlor-xanthon, 58 g Natriumcarbonat (getrocknet und gemahlen), 8,4 g Natriumhydrogencarbonat, 5 g Kaliumfluorid (spraygetrocknet), 600 g Diphenylsulfon, 500 g Xanthon. Der Kesselinhalt wurde zuerst auf 200 °C erhitzt. Nach 30 Minuten bei 200 °C wurde die Temperatur im Verlauf von 1 Stunde auf 320 °C gesteigert. Mit zunehmender Viskosität ändert sich die Farbe der Reaktionslösung von anfänglich Orange nach grau. Anschließend wurden 5 g 1,4-Bis(4-fluorbenzoyl)benzol als Kettenabbrecher zugegeben und die Lösung noch 30 Minuten nachgerührt.

Die viskose Reaktionslösung wurde auf einem Blech ausgegossen, die nach dem Erkalten erhaltene dünne Platte eingemahlen und daraus das Polymer wie folgt isoliert: 200 g Reaktionsmischung wurden jeweils für 1 Stunde extrahiert:
einmal mit 1,5 l Aceton bei 25 °C, zweimal mit 1,5 l Aceton bei Rückflußtemperatur,
dreimal mit 1,5 l Wasser bei 80 °C, einmal mit 1,5 l Aceton bei Rückflußtemperatur. Anschließend wurde bei 120 °C unter vermindertem Druck bis zur Gewichtskonstanz getrocknet. Ausbeute 83 %. Das Polymer zeigte bei DSC-Messungen eine Schmelztemperatur von 460 °C, die inhärente Viskosität betrug 0,81 dl/g.

4) Versuchsapparatur: 250-ml-Dreihalskolben, ausgestattet mit Heizpilz, Einlaß für Stickstoff-Schutzgas, Glasrührer, Innenthermometer, Abgasrohr mit Tauchung in Wasser (Blasenzähler). Die Messung der Viskosität der Reaktionslösung erfolgte durch Messung des Drehmoments an der Rührwelle.
Während des gesamten Reaktionsverlaufs wurde ein stetiger Stickstoff-Gasstrom über die Reaktionsmischung geleitet, um das bei der Reaktion entstehende Wasser zu entfernen.

In das Reaktionsgefäß wurden eingewogen: 13,23 g 2,5-Dihydroxy-2',4'-dichlorbenzophenon, 5,82 g Natriumcarbonat (getrocknet und gemahlen), 0,55 g Kaliumhydrogencarbonat, 1 g Kaliumfluorid (spraygetrocknet), 120 g Diphenylsulfon/Xanthon (1:1). Die Mischung wurde unter Rühren für 1 Stunden auf 200 °C erhitzt, die Temperatur im Verlauf von 1 Stunde auf 320 °C gesteigert und weitere 3 Stunden

bei dieser Temperatur gerührt. Anschließend wurden 0,5 g 4,4'-Dichlordiphenylsulfon als Kettenabbrecher zugegeben und die Lösung noch 20 Minuten nachgerührt. Die Aufarbeitung der Reaktionslösung erfolgt analog zu Beispiel 1, wobei das erhaltene Polymer die gleichen Eigenschaften wie in Beispiel 1 zeigte.

5 bis 7) In der nachfolgenden Tabelle sind weitere Beispiele angeführt, die analog Beispiel 3 bzw. 4 durchgeführt wurden.

**Ergänzung Polymerbeispiele**

| Beispiel | Monomer | Reaktions-bedingungen | Polymereigen-schaften |
|---|---|---|---|
| 5 | 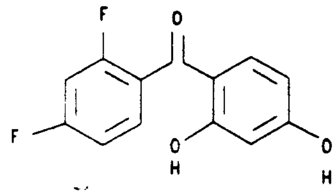 | Beispiel 3<br>Base: $K_2CO_3$<br>Lösemittel:<br>Xanthon | I.V. = 0.41<br>Kein Auf-schmelzen unter 400°C |
| 6 | 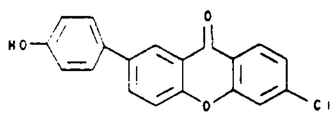 | Beispiel 4<br>Base: $Na_2CO_3$, KF<br>Lösemittel:<br>Xanthon | Kein Auf-schmelzen unter 400°C |
| 7 | 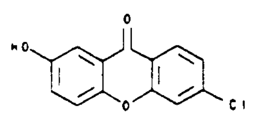<br>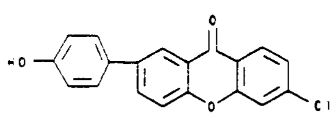<br>Mischung 1:1 | Beispiel 4<br>Base: $Na_2CO_3$, KF<br>Lösemittel:<br>Benzophenon | I.V. = 0.45 |

**Patentansprüche**

1. Polymere und Copolymere mit wiederkehrenden Einheiten der Formel (Ia) und (Ib)

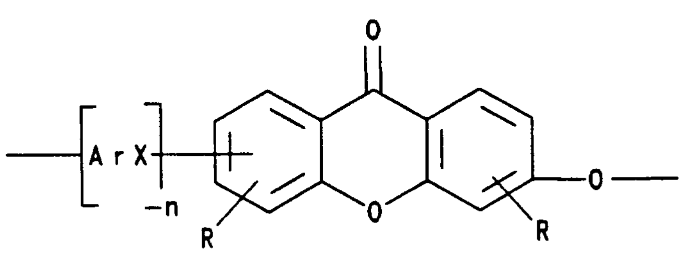

(Ia)

und/oder

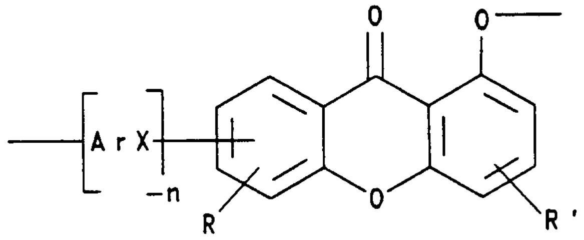

(Ib)

worin
R und R' gleich oder verschieden sind und Wasserstoff, Alkyl-, Alkoxy-, Aryl-, Aryloxy-Gruppen, benzanellierte Reste bedeuten,
Ar ist ein divalenter aromatischer Rest,
X eine direkte Bindung, -O- oder -S- und
n Null oder eins.

2. Polymere und Copolymere nach Anspruch 1, dadurch gekennzeichnet, daß R und R' Wasserstoff, Methyl-, Phenyl-, Methoxy-, Phenoxy-Gruppen sind und Ar eine Phenylen-Gruppe bedeutet.

3. Verfahren zur Herstellung von Polymeren und Copolymeren mit wiederkehrenden Einheiten der Formel (Ia) und/oder (Ib), dadurch gekennzeichnet, daß eine nucleophile Polykondensation unter im wesentlichen wasserfreien Bedingungen in Anwesenheit oder Abwesenheit eines polaren, apotischen aromatischen Lösemittels (i) wenigstens eines Hydroxyhalogen-substituierten Xanthons der Formel (IIa) oder (IIb)

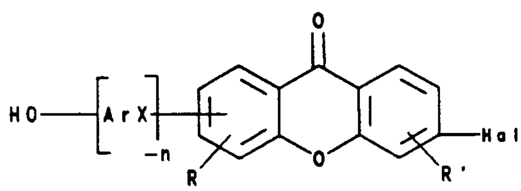

(IIa)

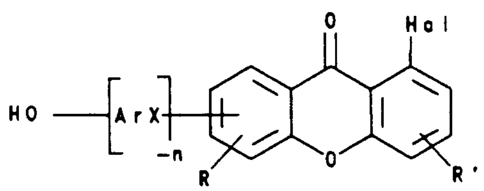

(IIb)

in welcher
R, R', Ar, X und n die in Anspruch 1 genannte Bedeutung haben und
Hal für Fluor oder Chlor steht, durchgeführt wird.

4. Verfahren zur Herstellung von Polymeren und Copolymeren mit wiederkehrenden Einheiten der Formel (Ia) und/oder (Ib), dadurch gekennzeichnet, daß eine nucleophile Polykondensation unter im wesentlichen wasserfreien Bedingungen in Anwesenheit oder Abwesenheit eines polaren, apotischen aromatischen Losemittels (ii) wenigstens einer Hydroxyhalogen-substituierten Benzophenon-Verbindung der Formel (IIIa) oder (IIIb)

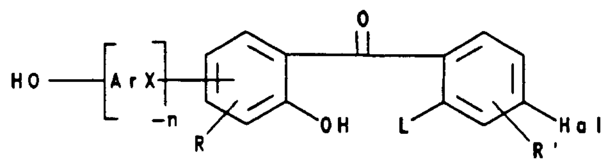

(IIIa)

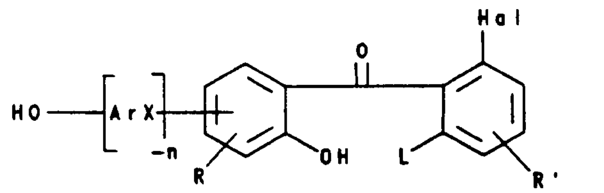

(IIIb)

worin
R, R', Ar, X, Hal und n die in Anspruch 3 genannte Bedeutung haben und
L eine durch intramolekulare nucleophile Substitution verdrängbare Fluchtgruppe bedeutet, durchgeführt wird.

**5.** Verfahren nach Anspruch 3 oder 4, dadurch gekennzeichnet, daß die Polykondensation wenigstens einer Hydroxy-halogen-Verbindung der Formel IIa) oder IIb) zusammen mit wenigstens einer Hydroxy-halogen-Verbindung der Formel (IV)

HO - Ar' - Hal (IV)

worin
Ar' ein divalenter aromatischer Rest mit 12 bis 26 C-Atomen ist, der mindestens eine Keto- oder Sulfonyl-Gruppe enthält, die ortho- oder para-ständig zur Hal-Gruppe steht und Hal für eine Chlor- oder Fluor-Gruppe steht, die sich am äußersten aromatischen Kern befindet, durchgeführt wird.

**6.** Verfahren nach einem oder mehreren der Ansprüche 3 bis 5, dadurch gekennzeichnet, daß Hal eine Chlor- oder Fluorgruppe und L ebenfalls eine Chlor- oder Fluorgruppe, darstellt.

**7.** Verfahren nach einem oder mehreren der Ansprüche 3 bis 6, dadurch gekennzeichnet, daß die Polykondensation in Gegenwart von Carbonaten oder Hydrogencarbonaten des Natriums und Kaliums oder Mischungen daraus erfolgt.

Europäisches Patentamt

# EUROPÄISCHER RECHERCHENBERICHT

Nummer der Anmeldung

EP 93 10 8107

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X,D | DATABASE WPI<br>Derwent Publications Ltd., London, GB;<br>AN 85-132631(22)<br>& JP-A-60 071 635 (TOSHIBA) 23. April 1985<br>* Zusammenfassung * | 1-3,6 | C08G65/40<br>C07C49/83 |
| X | DATABASE WPI<br>Derwent Publications Ltd., London, GB;<br>AN 85-132632(22)<br>& JP-A-60 071 636 (TOSHIBA KK) 20. September 1985 | 1,2 | |
| A | * Zusammenfassung * | 5 | |
| A | CHEMICAL ABSTRACTS, vol. 77, no. 27, 1972, Columbus, Ohio, US;<br>abstract no. 164389z,<br>ROYER R. ET AL 'Reactions induced by pyridine hydrochloride'<br>Seite 389 ;<br>* Zusammenfassung *<br>& BULL. SOC. CHIM. FR.<br>Nr. 7, 1972,<br>Seiten 2948 - 2851<br>ROYER R. ET AL | 3-5 | **RECHERCHIERTE SACHGEBIETE (Int. Cl.5 )**<br>C08G |
| A | US-A-3 985 783 (FRANCIS JOHNSON ET AL)<br>* Spalte 1, Zeile 60 - Spalte 2, Zeile 45 * | 3,4,6 | |
| A | DATABASE WPI<br>Derwent Publications Ltd., London, GB;<br>AN 88-202963(29)<br>& JP-A-63 141 942 (ASAHI CHEMICAL IND KK.) 14. Juni 1988<br>* Zusammenfassung * | 1,5 | |

Der vorliegende Recherchenbericht wurde für alle Patentansprüche erstellt

| Recherchenort | Abschlußdatum der Recherche | Prüfer |
|---|---|---|
| DEN HAAG | 25 AUGUST 1993 | O'SULLIVAN T.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)